# NEUE EUROPÄISCHE PATENTSCHRIFT

(11) **EP 0 077 752 B2**
(45) Veröffentlichungstag und Bekanntmachung des Hinweises auf die Entscheidung über den Einspruch: **26.07.1995**
(45) Hinweis auf die Patenterteilung: 05.03.1986
(21) Anmeldenummer: 82730129.2
(22) Anmeldetag: 13.10.1982
(51) Int. Cl.: A61K 49/00

(54) **Flüssige Mischung zur Aufnahme und Stabilisierung von Gasbläschen zur Verwendung als Kontrastmittel für die Ultraschalldiagnostik und deren Herstellung**
Liquid mixture for absorbing and stabilizing gas bubbles to be used as contrast agent in ultrasonic diagnosis and process for its preparation
Mélange liquide pour l'absorption et la stabilisation de vésicules de gaz utilisée comme agent de contraste pour le diagnostic par ultrasons et son procédé de préparation

(30) Priorität: 16.10.1981 DE 3141641
(43) Veröffentlichungstag der Anmeldung: 27.04.1983
(73) Patentinhaber: SCHERING AKTIENGESELLSCHAFT, 13342 Berlin (DE)
(72) Erfinder: Hilmann, Jürgen, D-1000 Berlin 65 (DE); Hoffmann, Rolf-Rüdiger, Dr., D-1000 Berlin 19 (DE); Mützel, Wolfgang, Dr., D-1000 Berlin 45 (DE); Zimmermann, Ingfried, Dr., D-1000 Berlin 28 (DE)
(74) Vertreter: Lederer, Franz, Dr.

(56) Entgegenhaltungen:
- WO-A-80/02365
- US-A- 4 276 885
- CHEMICAL ABSTRACTS, Band 93, Nr. 9, 1. September 1980, Seite 312, Nr. 91493x, Columbus, Ohio, USA B.A. CARROLL et al.: "Gelatin encapsulated nitrogen microbubbles as ultrasonic contrast agents"
- CHEMICAL ABSTRACTS, Band 78, Nr. 13, 2. April 1973, Seite 225, Nr. 81839r, Columbus, Ohio, USA M.C. ZISKIN et al.: "Contrast agents for diagnostic ultrasound"
- Meltzer et al. Ultrasound in Med. & Biol. vol. 6 pp 263-269 1980.
- Roeland et al. Vehr. Deutsche Gesellschaft für Herz und Kreislaufforschung. vol. 47. pp 185-192. 1981
- Meltzer et al. Journal Clin. Ultrasound vol. 9 pp 127-131 1981.
- Ibid vol. 8 pp 121-127. 1980.
- Strauss & Kunkle . Science vol. 186 pp 443-444.1974.
- Bommer et al. Abstracts of the 54th scientific sessions. Circulation, 64. Abstract 770 1981.
- Kirk-Othmer Enc. of Chemical Technology, vol. 6 1965. s. 926-932.
- Pharmazeutische Technologie. Tenside,S.300-305. 1978.

## Beschreibung

Die Erfindung betrifft eine flüssige Zusammensetzung zur Aufnahme und Stabilisierung von mit physiologisch verträglichem Gas gefüllten Gasblaschen zur Verwendung als Kontrastmittel für die Ultraschalldiagnostik von mit Flüssigkeit gefüllten Gefäßen oder Hohlräumen des menschlichen und tierischen Körpers.

Es ist allgemein bekannt, daß der Kontrast in der Ultraschall-Diagnostik durch die Anwesenheit von Gasbläschen in der Flüssigkeit (Blut), die das Untersuchungsobjekt durchströmt, erhöht wird. Zu diesem Zweck kann man diese Gas-Bläschen außerhalb des Untersuchungsobjektes herstellen und in den Blutstrom injizieren. Beispielsweise ist dies möglich, indem man eine flüssige Lösung wie Kochsalzlösung, eine Farbstofflösung (Ziskin, Marvin C. et al., Invest. Radiol. 1972. 7-6. 500-505) oder vorher entnommenes Blut heftig schüttelt, um die Bläschen zu erzeugen, danach injiziert und die Ultraschall-Untersuchung durchführt. So berichtet Feigenbaum et al., in dem Artikel 〈〈 Identification of Ultrasound Echoes From the Left Ventricle of the Heart Through the Use of Injections of Indocyanine Green 〉〉 (Circulation, Volume XLI. April 1970) über die Erzeugung von Echos durch Gasbläschen im linken Ventrikel des Herzens, ähnlich wie auch von Gramiak et al. (Radiology 100. 415-418. 1971) berichtet wird. Ein anderes Verfahren zur Erzeugung von Mikro-Gasbläschen mit bestimmter Größe wird in dem Bericht 〈〈 Non-Invasive Assessment of Pulmonary Hypertension Using The Bubble Ultrasonic Resonance Pressure (BURP) Method 〉〉 (Report No. HR-62917-1A. Apr. 1977. Division of Lung Diseases, National Heart, Lung and Blood Institute) beschrieben.

In dem US-Patent Nr. 4 265 251 wird die Herstellung von mikro-Gasbläschen mit einer Saccharid-Hülle beschrieben, die durch die Verwendung einer aufwendigen komplizierten Apparatur mit reproduzierbarer Größenverteilung hergestellt werden können. Die Nachteile dieses Verfahrens liegen einmal darin, daß die mikro-Bläschen kurz vor der Verwendung mit dem Träger offen durchgemischt werden, wodurch Sterilität und Pyrogenfreiheit nicht gewährleistet sind. Zum anderen verursacht,die Herstellung durch die aufwendige Technik hohe Kosten. In einem weiteren US-Patent (4 276 885) und von Carroll. Barbara A., et al. in Invest. Radiol. 1980. 15 (3). 260-266 wird beispielsweise ein Verfahren beschrieben, mikro-Gasbläschen mit einer Gelatinemembrane herzustellen und als Träger für diese Mikro-Bläschen ein gelierbares Medium zu verwenden, damit die Gasbläschen durch Abkühlen eingefroren und bei Bedarf durch Erwärmen wieder in Freiheit gesetzt werden können.

Nachteilig bei dieser Methode ist die Tatsache, daß eine so erzeugte Suspension nicht sterilisiert werden kann, da bei Hitzesterilisation die Mikro-Bläschen nicht haltbar sind und auch durch Sterilfiltration abgetrennt oder zerstört werde Dazu kommt, daß Gelatinezubereitungen stets ein Anaphylaxie-Risiko darsteilen.

Durch die Bereitstellung des erfindungsgemäßen neuen Ultraschall-Kontrastmitels werden die geschilderten Nachteile beseitigt. Gegenstand der Erfindung ist eine flüssige Zusammensetzung gemäß Anspruch 1.

Die Herstellung der Bläschen kurz vor der Verwendung kann auf die verschiedenste Weise erfolgen:
1. Durch Mitaufziehen von Luft oder physiologisch verträglichem Gas beim Aufziehen der erfindungsgemäßen Mischung und mehrmaliger Wiederholung von Aufziehen und Ausspritzen sog. Pumpen) unter sterilen Bedingungen.
2. Durch zunächst getrenntes Herstellen und nachfolgende Hitzesterilisation der Mischung von Tensid in Wasser oder einer physiologisch verträglichen wäßrigen Lösung und der Mischung des viskositätserhöhenden Stoffes in Wasser oder einer physiologisch verträglichen wäßrigen Losung. Aufziehen der ersten Mischung und nachfolgendes Einspritzen dieser ersten Mischung in die zweite, die sich in einem steilen Gefäß zusammen mit einem physiologisch verträglichen Gas befindet.
3. Durch Aufbewahrung einer sterilen Mischung von Tensid und viskositätserhöhenden Stoff in Wasser oder einer physiologisch verträglichen wäßrigen Lösung in einer sterilen Atmosphäre eines physiologisch verträglichen Gases unter einem Druck, der größer als der Normaldruck ist, vorzugsweise in einem druckfest verschließbaren Gefäß mit einer Vorrichtung zum Ablassen des Überdrucks, indem man, gegebenenfalls nach vorherigem Schütteln zum besseren Vermischen des Gases mit der Lösung, den Überdruck entweichen läßt.

Das Vermischen der beiden flüssigen Mischungen kann mit jeder Methode erfolgen, durch die eine kräftige Verwirbelung erzielt wird, beispielsweise durch mechanisches Rühren oder durch Ultraschall oder durch Aufziehen der einen Mischung in eine Injektionsspritze und Entleeren dieser Spritze in die zweite Mischung unter Anwendung von möglichst hohem Druck und hoher Ausströmungsgeschwindigkeit und nachfolgendes kräftiges Schütteln, wobei für alle Mischungsvorgänge sterile Bedingungen gewährleistet sein müssen. Beispielsweise wird für den Mischvorgang ein Gefäß verwendet, das sterile Bedingungen ermöglicht und groß genug ist und nach der Aufnahme der zweiten Mischung für das nachfolgende kräftige Schütteln noch einen genügend großen Gasraum besitzt. Vorzugsweise verwendet man dafür genügend große Multivials mit einem Verschluß, der mit einer Injektionsnadel durchstochen werden kann und so das Einspritzen der Mischung, das nachfolgende Durchmischen und die Entnahme der mit Bläschen versehenen flüssigen Mischung ohne Öffnen erlaubt.

Falls erforderlich, kennen die Bläschen anstelle mit steriler Luft mit anderen physiologisch verträglichen sterilen Gasen wie beispielsweise Kohlendioxid. Sauersoff, Stickstoff, Edelgasen oder mit deren Gemischen gefüllt sein, wobei sterile Luft. Kohlendioxid und oder Sauerstoff bevorzugt sind. Zu diesem Zweck wird aus der Mischung des Tensides oder Tensidgemisches und oder aus der Mischung der viskositatserhöhenden Substanz durch Begasung mit dem gewünschten Gas die Luft verdrängt und beide Mischungen in einer der vorbeschriebenen Weisen in einem Multivial gemischt, das mit dem gewünschten Gas oder Gasgemisch gefüllt ist.

Nach der Erzeugung der Bläschen in einer der angegebenen oder äquivalenten Verfahrensweise sind die Mischungen zur intravenösen Verabreichung als Kontrastmittel für die Ultraschalldiagnostik vorbereitet.

Es steht damit ein Kontrastmittel für die Ultraschalldiagnostik zur Verfügung, das frei ist von festen Partikeln und steril ist.

Die Tenside sind nichtionogene Tenside aus der Gruppe: Lecithine, Lecithinfraktionen und deren Abwandlungsprodukte, Polyoxyethylenfettsäureester, Polyoxyethylenfettalkoholäther, ethoxylierte Rizinusöle und deren hydrierte Derivate, Polyoxyethylenpolyoxypropylen-Polymere, wobei Polyoxyäthylenfettsäurestearate und Polyoxyethylenpolyoxypropylen-Polymere mit dem Molgewicht 6 800-8 975. 13 300 und 16 250 bevorzugt sind. Sämtliche Prozentangaben beziehen sich auf Gewichtsprozente.

Die Kontrastmittel-Lösung enthält erfindungsgemäß mindestens 0,01% eines Tensides oder des Gemisches mehrerer Tenside, wobei der bevorzugte Gehalt 0,5 bis 5% Tensid oder Tensidgemisch beträgt.

Die viskositätserhöhenden Substanzen sind Polysaccharide, Lactose, Saccharose, Dextrane, Cyclodextrine, Hydroxyäthylstärke, Plasmaproteine, Gelatine, Oxypolygelatine und Gelatine derivate oder deren Gemische sowie Polyoxyethylenpolyoxypropylen-Polymere mit einem Molekulargewicht von 4 750-16 250.

Die Konzentration dieser genannten Stoffe in der Lösung beträgt erfindungsgemäß mindestens 0,5%, wobei die Höchstkonzentration auch vom gelösten Stoff abhängt. So kann beispielsweise Gelatine mit einer Konzentration von 0,5 bis 2% verwendet werden. Die Oxypolygelatine wird bevorzugt mit einer Konzentration von 0,5 bis 10% eingesetzt.

Man kann auch Tenside verwenden, die gleichzeitig viskositätserhöhend wirken wie Polyoxyethylenpolyoxypropylen-Polymere mit dem Molekulargewicht von 4 750 bis 16 250. In diesem Fall beträgt die Konzentration der Tenside mit viskositätserhöhender Wirkung in der Gesamtlösung 1% bis 10%, vorzugsweise 3% bis 10%. Das Tensid oder Tensidgemisch wird vorzugsweise in Gegenwart des viskositätserhöhenden Stoffes oder Stoffgemisches in Wasser oder einer physiologisch verträglichen wäßrigen Lösung gelöst. Als physiologisch verträgliche wäßrige Lösung können beispielsweise physiologische Elektrolytlösungen wie physiologische Kochsalzlösung, Ringerlösung oder die wäßrigen Lösungen von Natriumchlorid, Kalziumchlorid, Natriumhydrogencarbonat, Natriumcitrat, Natriumazetat oder Natriumtartrat oder Salzlösungen (Infusionslösungen) verwendet werden.

So werden beispielsweise für die Ultraschall-Kontrastaufnahme der rechten Herzkammer beim Hund (Beagle Hunde von 17.2 kg Körpergewicht, 2.5 Jahre alt, männlich, geschlossener Thorax) 0.3 ml einer Bläschen-Suspension verwendet, die durch starkes Verwirbeln von 2 ml 5 %iger wäßriger sterilisierter Pluronic ^{(R)} 68-Lösung und 8 ml wäßriger sterilisierter 5 %iger Glucose-Lösung in einer sterilen Luftatmosphäre erzeugt wurde. Zur Verwirbelung wurde die Pluronic^{(R)} F 68-Lösung mit einer Injektionsspritze aufgezogen, diese Lösung in ein Multivial mit der Glucoselösung mit hoher Ausströmungsgeschwindigkeit gespritzt und anschließend geschüttelt. Die Größenverteilung der Gasbläschen wurde 2 Minuten nach der Herstellung mit einem Cilas-Granulometer 715 bestimmt und betrug für 50 % der Gasbläschen 35 µm. Die Sichtbarmachung und Aufzeichnung der Ultraschall-Echos sowie deren diagnostische Auswertung erfolgt in an sich bekannter Weise und ist zum Beispiel in USP 4 276 885 sowie von H. L. Wyatt et al. in Circulation 60. S. 1 104 f (1979) beschrieben.

### Beispiel 1

Einer 10 %igen wäßrigen und sterilen Pluronic ^{(R)} F 127-Lösung werden 2 ml mit einer Injektionsspritze entnommen und mit möglichst hoher Ausströmungsgeschwindigkeit in 8 ml einer sterilen physiologischen Kochsalzlösung, die sich in einem sterilen 25 ml Multivial unter einer Luftatmosphäre befinden, gespritzt. Anschließend wird die Mischung stark geschüttelt. Die hergestellte Gasblasensuspension enthalt 2 % Pluronic ^{(R)} F 127 und 0.9 % Kochsalz. Die mit einem modifizierten Cilas-Granulomter 2 Minuten nach der Herstellung ermittelte durchschnittliche Bläschengroße beträgt für 50 % < 45 µm.

### Beispiel 2

Einer wäßrigen und sterilen 5 %igen Pluronic ^{(R)} F 68-Lösung, aus der vorher die gelöste Luft mit Argon verdrängt worden ist, werden 2 ml mit einer Injektionsspritze entnommen und mit möglichst hoher Ausströmungsgeschwindigkeit in 8 ml einer sterilen, wäßrigen 6 %igen Dextran 40-Lösung, aus der ebenfalls die geloste Luft mit Argon verdrängt worden ist und die sich in einem sterilen 25 ml Multivial unter ein Argon-Atmosphäre befinden, gespritzt Anschließend wird die Mischung stark geschüttelt. Die mit dem Cilas-Granulometer 715 2 Minuten nach der Herstellung ermittelte Größenverteilung der Argon-Gasblasen ergab für 50 % < 55 µm. Die erhaltene Gasblasensuspension enthält 1 % Pluronic ^{(R)} F 68 und 4.8 % Dextran 40.

### Beispiel 3

Analog Beispiel 2, jedoch unter Verwendung von Helium anstelle von Argon, erhält man nach dem Mischvorgang eine Helium-Gasblasensuspension, die 1 % Pluronic ^{(R)} F 68 und 4.8 % Dextran 40 enthält.

## Patentansprüche

1. Flüssige Zusammensetzung zur Aufnahme und Stabilisierung von mit physiologisch verträglichem Gas gefüllten Gasbläschen zur Verwendung als Kontrastmittel für die Ultraschalldiagnostik bestehend aus einer Mischung von
a) 0,01-10 Gew.% eines oder mehrerer nichtionogene Tenside aus der Gruppe Lecithine, Lecithinfraktionen und deren Abwandlungsprodukte, Polyoxyethylenfettsäureester, Polyoxyethylenfettalkoholäther, ethoxylierte Rizinusöle und deren hydrierte Derivate, Polyoxyethylenpolyoxypropylen-Polymere, mit Wasser oder einer physiologisch verträglichen wäßrigen Lösung.
und einer Mischung von
b) 0,5-50 Gew.% einer oder mehrerer viskositätserhöhenden Substanzen aus der Gruppe Lactose, Saccharose, Polysaccharide, Dextrane, Cyclodextrine, Hydroxyäthylstärke, Plasmaproteine, Gelatine, Oxypolygelatine und andere Gelatinederivate sowie Polyoxyethylenpolyoxypropylen-Polymere mit einem Molekulargewicht von 4.750-16.250, in Wasser oder einer physiologisch verträglichen wäßrigen Lösung mit der Maßgabe, daß die Gesamtlösung mindestens 0,01, vorzugsweise 0,5-5 Gew.% des Tensids und mindestens 0,5 Gew.% der viskositätserhöhenden Substanz enthält, sowie mit der weiteren Maßgabe, daß bei Verwendung von Polyoxyethylenpolyoxypropylen-Polymer mit dem Molekulargewicht von 4.750-16.250 als Tensid, welches gleichzeitig viskositätserhöhend wirkt, dessen Konzentration in der Gesamtlösung 1-10%, vorzugsweise 3-10% beträgt.

2. Flüssige Zusammensetzung gemäß Anspruch 1, dadurch gekennzeichnet, daß sie als Tensid das Polyoxyethylenpolyoxypropylen-Polymere mit dem Molekulargewicht 6.800 bis 8.975 (Pluronic ^{(R)} F 68) oder 16.250 (Pluronic ^{(R)} F 108) oder 13.300 (Pluronic ^{(R)} F 127) enthält.

3. Flüssige Zusammensetzung gemäß Anspruch 1, dadurch gekennzeichnet, daß sie als Tensid Polyoxyethylenfettsäurestearat enthält.

4. Flüssige Zusammensetzung gemäß einem der Ansprüche 1-3, dadurch gekennzeichnet, daß sie als physiologisch verträgliche wäßrige Lösung die wäßrige Lösung eines ein- oder mehrwertigen Alkohols, physiologische Elektrolytlösung, Infusionslösung oder deren Gemische enthält.

5. Flüssige Zusammensetzung gemäß einem der Ansprüche 1-4 zur Aufnahme und Stabilisierung von mit steriler Luft, Kohlendioxid, Sauerstoff, Stickstoff mit einem Edelgas oder deren Gemischen gefüllten Gasbläschen.

## Claims

1. Liquid composition for the uptake and stabilisation of gas bubbles filled with physiologically compatible gas for use as contrast medium in ultra sound diagnosis, consisting of a mixture of
a) from 0.01 to 10% by weight of one or more non-ionic surfactants from the group consisting of lecithins, lecithin fractions and derivatives thereof, polyoxyethylene fatty acid esters, polyoxyethylene fatty alcohol ethers, ethoxylated castor oils and hydrogenated derivatives thereof and polyoxyethylenepolyoxypropylene polymers, with water or with a physiologically compatible aqueous solution,
and of a mixture of
b) from 0.5 to 50% by weight of one or more viscosity-increasing substances from the group consisting of lactose, saccharose, polysaccharides, dextrans, cyclodextrins, hydroxyethyl starch, plasma proteins, gelatin, oxypolygelatin and other gelatin derivatives, and polyoxyethylenepolyoxypropylene polymers having a molecular weight of from 4 750 to 16 250, in water or in a physiologically compatible aqueous solution,
with the proviso that the total solution contains at least 0.01% by weight, preferably from 0.5 to 5% by weight, of the surfactant and at least 0.5% by weight of the viscosity-increasing substance, and with the further proviso that when there is used as surfactant a polyoxyethylenepolyoxypropylene polymer having a molecular weight of from 4 750 to 16 250 that simultaneously has a viscosity-increasing effect, the concentration thereof in the total solution is from 1 to 10%, preferably from 3 to 10%.

2. Liquid composition according to claim 1, characterised in that as surfactant it contains a polyoxyethylenepolyoxypropylene polymer with a molecular weight of from 6 800 to 8 975 (Pluronic ^{(R)} F 68) or of 16 250 (Pluronic ^{(R)} F 108) or of 13 300 (Pluronic ^{(R)} F 127).

3. Liquid composition according to claim 1, characterised in that as surfactant it contains polyoxyethylene fatty acid stearate.

4. Liquid composition according to any one of claims 1 to 3, characterized in that it contains as physiologically compatible aqueous solution the aqueous solution of a univalent or polyvalent alcohol, physiological electrolyte solution, infusion solution, or mixtures thereof.

5. Liquid composition according to any one of claims 1 to 4, for the uptake and stabilization of gas bubbles filled with sterile air, carbon dioxide, oxygen, nitrogen, a noble gas, or mixtures thereof.

## Revendications

1. Composition liquide pour la réception et la stabilisation de petites bulles de gaz remplies d'un gaz physiologiquement acceptable pour l'utilisation comme agent de contraste pour le diagnostic échographique, composé
a) de 0,01 à 10 % en poids d'un ou plusieurs agents tensio-actifs non ionogènes pris dans le groupe comportant : les lécithines, les fractions de lécithine et leurs produits de modification, les esters polyoxyéthylèniques d'acides gras, les éthers polyoxyéthylèniques d'alcools gras, les huiles de ricin éthoxylées et leurs dérivés hydrogénés, les polymères de polyoxyéthylène-polyoxypropylène, avec l'eau ou une solution aqueuse physiologiquement acceptable.
et d'un mélange
b) de 0,5 à 50% en poids d'une ou plusieurs substances augmentant la viscosité prise dans le groupe comportant : le lactosee, le saccharose, les polysaccharides, les dextranes, les cyclodextrines, les hydroxyéthylamidons, les protéines plasmatiques, la gélatine, la polyoxygélatine et autres dérivés de gélatine ainsi que des polymères de polyoxyéthylène-polyoxypropylène avec un poids moléculaire de 4 750 à 16 250, dans l'eau ou une solution aqueuse physiologiquement acceptable,
à la condition que la totalité de la solution contienne au moins 0,01, de préférence de 0,5 à 5% en poids de l'agent tensio-actif et au moins 0,5 % en poids de la substance augmentant la viscoté, ainsi qu'à la condition supplémentaire que dans l'utilisation du polymère de polyoxyéthylène-polyoxypropylène avec un poids moléculairee de 4 750 à 16 250 comme agent tensio-actif, qui agit en même temps comme agent augmentant la viscosité, dont la teneur dans la totalité de la solution est de 1 à 10%, de préférence de 3 à 10%.

2. Composition liquide selon la revendication 1, caractérisée en ce qu'elle contient comme agent tensio-actif le polymère de polyoxyéthylène-polyoxypropylène avec un poids moléculaire de 6 800 à 8 975 (Pluronic® F 68) ou 16 250 (Pluronic® F 108) ou 13 300 (Pluronic® F 127).

3. Compositiuon liquide selon la revendication 1, caractérisée en ce qu'elle contient comme agent tensio-actif le stéarate d'acide gras-polyoxyéthylène.

4. Composition liquide selon l'une des revendications 1 à 3, caractérisée en ce qu'elle contient comme solution aqueuse physiologiquement acceptable, la solution aqueuse d'un alcool mono- ou polyfonctionnel, une solution d'électrolyte physiologique, une solution de perfusion ou leurs mélanges.

5. Composition liquide selon l'une des revendications 1 à 4 pour la réception et la stabilisation de petites bulles de gaz remplies de l'air, de l'anhydride carbonique, de l'oxygène, de l'azote stérile avec un gaz noble ou leurs mélanges.
